# EUROPEAN PATENT SPECIFICATION

(11) **EP 1 301 222 B1**
(45) Date of publication and mention of the grant of the patent: **14.12.2005**
(21) Application number: 01953563.2
(22) Date of filing: 19.07.2001
(51) Int. Cl.: A61L 27/36, A61L 31/00, A61F 2/28, A61F 2/44

(54) **OSTEOIMPLANT AND METHOD OF MAKING SAME**
OSTEOIMPLANTAT UND VERFAHREN ZU SEINER HERSTELLUNG
IMPLANT OSSEUX ET SON PROCEDE DE FABRICATION

(30) Priority: 19.07.2000 US 219198 P
(43) Date of publication of application: 16.04.2003
(73) Proprietor: Osteotech, Inc., Eatontown, NJ 07724 (US)
(72) Inventor: EDWARDS, Jean, T., Hillsborough, NJ 08844 (US); SCARBOROUGH, Nelson, L., Andover, MA 01810 (US); MANRIQUE, Albert, Manalapan, NJ 07726 (US); BODEN, Scott, D., Atlanta, GA 30033 (US)
(74) Representative: Zinnecker, Armin
(86) International application number: PCT/US2001/022853
(87) International publication number: WO 2002/005750

(56) References cited:
- WO-A-00/50102
- WO-A-97/25941
- WO-A-99/39757
- US-A- 5 507 813

## Description

### BACKGROUND OF THE INVENTION

### 1. Field of the Invention

This invention relates to a porous three-dimensional osteoimplant comprising a low density coherent matrix of bone particles and to a method for making the osteoimplant. The osteoimplant maintains its shape and cohesiveness upon absorption of fluid. The porous, absorbent osteoimplant of the invention can possess any desired shape, e.g., square or rectangular blocks, cylinders, wedges, and the like, and in accordance with a preferred embodiment, is provided with one or more cavities which can be filled with an osteogenic material which promotes and/or accelerates new bone formation at the site of implantation. Such cavities prevent the loss or migration of the osteogenic material away from the implantation site.

### 2. Description of the Related Art

Shaped or cut bone segments that can optionally be rendered to be osteoinductive via demineralization have been used extensively to solve various medical problems in human and animal orthopedic surgical practice and their application has also extended to the fields of, e.g., cosmetic and reconstructive surgery, dental reconstructive surgery, podiatry, orthopaedics, neurosurgery and other medical fields involving surgery of hard tissues. The use of autograft bone (where the patient provides the source), allograft bone (where another individual of the same species provides the source) or xenograft bone (where another individual of a different species provides the source) is well known in both human and veterinary medicine. In particular, transplanted bone is known to provide support, promote healing, fill bony cavities, separate bony elements (such as vertebral bodies), promote fusion (where bones are induced to grow together into a single, solid matrix), or stabilize the sites of fractures. More recently, processed bone has been developed into shapes for use in new surgical applications, or as new materials for implants that were historically made of non-biologically derived materials.

Allograft bone is known to have osteoconductive and osteoinductive capabilities, although the osteoinductive properties are limited because of the necessary tissue sterilizing and cleaning procedures associated with harvesting these bone grafts. The term osteoconduction refers to the capability of a three-dimensional material to conduct the ingrowth of new living bone into and around its structure. The term osteoinduction refers to the capability of recruiting pluripotent cells of the patient and promoting their differentiation into osteoblasts, which are bone forming cells. An osteoinductive material will typically form bone if implanted into living tissue where bone would not normally be found. For example, the placement of demineralized bone powder into the muscle of a patient will result in ectopic (outside of bone) bone formation.

U.S. Patent No. 5,507,813 describes a surgically implantable sheet formed from elongate bone particles, optionally those that have been demineralized. The sheet may further contain biocompatible ingredients, adhesives, fillers, plasticizers, etc. The osteoinductive sheet is rigid and relatively strong when dry and flexible and pliable when wetted or hydrated. These sheets are sold under the tradename Grafton®Flex (Osteotech, Inc., Eatontown, New Jersey, USA). These sheets must be wetted/hydrated prior to use in order to render the dense matted sheets useful for implantation.

U.S. Patent No. 4,932,973 describes an artificial organic bone matrix with holes or perforations extending into the organic bone matrix. The holes or perforations are indicated to be centers of cartilage and bone induction following implantation of the bone matrix into living tissue.

U.S. Patent No. 4,394,370 discloses a one-piece sponge-like bone graft material fabricated from fully demineralized bone powder or microparticulate bone, and reconstituted collagen. The sponge-like graft is optionally crosslinked with glutaraldehyde.

Another one-piece porous implant is described in U.S. Patent No. 5,683,459. The implant is made up of a biodegradable polymeric macrostructure composed of chemotactic ground substances such as hyaluronic acid.

WO 97 25941 A discloses an osteoimplant fabricated from a porous matrix of elongate demineralized bone particles.

US-A-5 507 813 discloses an osteoimplant comprising a porous matrix made of elongate demineralized bone particles and a process for making such materials for an application to the site of a bone defect, leading to new bone ingrowth.

WO 99 39757 A discloses an osteoimplant made of a porous matrix of demineralized bone particles with adjacent bone-derived elements being bonded to each other through chemical linkages.

### SUMMARY OF THE INVENTION

It is an object of the invention to provide an osteoimplant which exhibits both osteoconductive and osteoinductive properties.

It is an object of the invention to provide an osteoimplant fabricated from a low density, coherent, three-dimensional matrix of demineralized bone particles wherein the osteoimplant is capable of being formed into a wide variety of shapes not limited by the original shape of the bone(s) from which the particles are derived.

It is an object of the invention to provide a low density osteoimplant which possesses an open pore structure which allows the osteoimplant to readily absorb fluids such as blood and yet still retain its original shape.

It is an object of the invention to provide a low density osteoimplant fabricated from bone particles which is flexible when dry and which may be implanted while in the dry state.

It is a further object of the invention to provide a method of making a low density osteoimplant possessing the aforementioned characteristics.

It is yet an even further object of the invention to provide a method of repairing a bone defect which utilizes a low density osteoimplant possessing the aforementioned characteristics.

These and other objects of the invention are met by an osteoimplant which comprises a shaped, coherent, three-dimensional porous matrix of elongate demineralized bone particles, wherein said matrix possesses a bulk density of lower than 0.3 g/cm³. The open pore structure of the osteoimplant of the invention is highly absorbent and sponge-like in nature. The osteoimplant is flexible when dry (i.e., when containing less than about 5 weight percent water) and does not require time consuming rehydration prior to implantation. It may assume any desired shape and/or configuration and may be cut, e.g., with surgical scissors, before and/or after the implant has absorbed fluid. Even in the wetted/hydrated state, the osteoimplant of the invention maintains its original shape and coherency, and can be handled with ease by the medical practitioner. The osteoimplant of the invention represents a significant advance in the field of bone grafts because due to its low density and open pore structure it is both highly osteoconductive and functionally versatile and due to its demineralized bone content it exhibits excellent osteoinductivity. Osteoinductivity can be conveniently quantified as the amount of bone formed in an etopic site in an athymic nude rat Scores are rated 0 to 4. The osteoimplants of the invention exhibit osteoinductivities of at least 2, typically greater than 3, when measured in an athymic rat assay as described in Edwards JT, Diegmann MH, Scarborough NL, Osteoinduction of Human Demineralized Bone: Characterization in an Animal Model, Clin. Orthop. Rel. Res. 357:219 228 (1998) (described in detail in Example 4 hereinbelow).

The osteoimplant of the invention can be combined with a wide variety of biocompatible substances which can be introduced into the porous matrix of the osteoimplant and/or into large cavities, depressions, and the like, produced in the osteoimplant. Thus, the implant herein functions as a highly effective carrier and/or delivery vehicle for bone-growth inducing and/or otherwise medically useful substances.

Further provided herein is a method of fabricating the osteoimplant herein which comprises providing a quantity of elongate demineralized bone particles, mixing the elongate demineralized bone particles with a wetting agent comprising water to provide a liquid composition containing from 5 to 40 volume percent swollen, hydrated bone particles, placing the liquid composition in a mold, heating the liquid composition in the substantial absence of pressure at a temperature above 35°C for a period of time sufficient to remove water present in the wetting agent to provide an osteoimplant comprising a shaped, coherent, three-dimensional porous matrix of elongate demineralized bone particles wherein said matrix possesses a bulk density of lower than 0.3 g/cm³.

Further provided in accordance with the invention is a method of repairing and/or treating bone comprising implanting at a bone repair site an osteoimplant which comprises a shaped, coherent, three-dimensional porous matrix of elongate demineralized bone particles, wherein said matrix possesses a bulk density of lower than 0.3 g/cm³. The osteoimplant of the invention can be applied to virtually any bone repair site in the body and can be utilized alone or in combination with one or more adjunct medical devices and/or procedures. The osteoimplant of the invention finds particular utility in the areas of dental reconstructive surgery and spinal fusion where substantial amounts of body fluid, e.g., saliva and/or blood, are frequently encountered, or where autograft (e.g., local bone, marrow or iliac crest, etc.) is incorporated in the osteoimplant. The unique ability of the low density porous osteogenic implant to absorb such body fluids and yet still retain its original shape represents a significant advance in the medical field.

### BRIEF DESCRIPTION OF THE DRAWINGS

Fig. 1 is a photograph which depicts several non-limiting embodiments of the osteoimplant of the invention in various shapes and sizes;
Fig. 2 is a photograph of several osteoimplants of the invention possessing generally cylindrical configuration;
Fig. 3 is a photograph of an osteoimplant produced in accordance with the teachings of the present disclosure wherein the implant is provided with a preformed cavity or depression;
Fig. 4 generally depicts a mold which can be utilized in the fabrication of an osteoimplant such as that depicted in Fig. 3.

### DESCRIPTION OF THE PREFERRED EMBODIMENTS

In order to better understand the disclosure herein, including the claims and the various figures, the following is a partial glossary of terms and expressions intended to be non-limiting and understood in their broadest sense. Such terms and expressions are also intended to refer to any and all phrases of like import. Definitions of terms and expressions utilized herein are also found elsewhere in this disclosure and may not be present in the glossary of terms below.

The term "osteoimplant" as utilized herein is intended to refer to any device or material for implantation in living tissue that aids or augments bone formation or healing, including the induction of bone formation in soft tissue, or within other implant devices such as spinal cages. Osteoimplants are most often applied at a bone defect site, e.g., one resulting from injury, defect brought about during the course of surgery, infection, malignancy or developmental malformation. Therefore, such "osteoimplants" are envisioned as being suitably sized and shaped as required for use in a wide variety of orthopedic, neurosurgical, and oral and maxillofacial surgical procedures such as the repair of simple and compound fractures and non-unions, external and internal fixations, joint reconstructions such as arthrodesis, general arthroplasty, deficit filling, discectomy, laminectomy, anterior cervical and thoracic operations, spinal fusions, etc. Therefore, the osteoimplants utilized herein are intended for implantation at a bony site and are made of elongate demineralized bone particles optionally in combination with any biocompatible material(s), e.g., bone or bone particles not possessing an elongate configuration, biocompatible synthetic materials, combinations thereof, etc, and may be designed for either animal or human use. The term "osteoimplant" herein is therefore utilized in its broadest sense and is not intended to be limited to any particular shapes, sizes, configurations, or applications.

The term "biocompatible" and expressions of like import shall be understood to refer to those materials which elicit no unacceptable detrimental biological responses in the recipient in which they are implanted. Thus, implants or osteoimplants which elicit acceptable, mild, transient inflammation and/or granulation responses are considered biocompatible.

The term "osteogenic" as utilized herein shall be understood as referring to the ability of an osteoimplant to enhance or accelerate the growth of new bone tissue by one or more mechanisms such as osteogenesis, osteoconduction and or osteoinduction.

The term "shaped" as applied to the matrix of elongate demineralized bone particles herein refers to a determined or regular form or configuration, in contrast to an indeterminate or vague form or configuration (as in the case of a lump or other solid matrix of no special form) and is characteristic of such materials as sheets, plates, disks, cones, pins, screws, tubes, teeth, bones, portion of bone, wedges, cylinders, threaded cylinders, and the like, as well as more complex geometric configurations.

The term "coherent" as applied to the matrix of elongate demineralized bone particles refers to the ability of the bone particles to adhere to each other either mechanically, e.g., by entanglement, or by use of a biocompatible binder or adhesive whether the shaped material is in the dry or wetted, e.g., hydrated, state.

The expression "three-dimensional" refers to the ability of the matrix of elongate demineralized bone particles to assume any desired shape and/or configuration.

The expression "open pore structure" as it applies to the matrix of elongate demineralized bone particles shall be understood as referring to the low density absorbent sponge-like nature of the matrix in which there are a plurality of accessible pores or openings which are present throughout the entire volume of the matrix.

The term "incorporation" utilized herein refers to the biological mechanism whereby host tissue gradually replaces the osteoimplant of the invention with native host bone tissue. This phenomenon is also known in the scientific literature as "bone remodeling" or "cellular based remodeling" and "wound healing response". Therefore, the term "incorporation" utilized herein shall be understood as embracing what is known by those skilled in the art as the various expressions set forth above.

The expression "further treatment" as utilized herein refers to procedures such as, e.g., lyophilizing, cross-linking treatment, re-mineralization, sterilization, etc., performed either before, during or after the step of heating the liquid composition as well as post process procedures such as, e.g., machining, laser etching, welding, assembling of parts, cutting, milling, reactive etching, etc.

The osteoimplant of this invention comprises a coherent matrix of elongate demineralized bone particles possessing a bulk density of less than 0.3 g/cm³. The elongate demineralized bone particles form a continuous three-dimensional matrix possessing an open pore structure. The matrix readily absorbs fluids, such as body fluids (e.g. blood or marrow) into its void volume. Upon absorption of fluids, the porous osteoimplant maintains its shape and cohesiveness. Elongate bone particles utilized herein possess relatively high medium length to medium thickness ratios. Such elongate bone particles can be readily obtained by any one of several methods, e.g., by milling, shaving or planing the surface of an entire bone or relatively large section of bone. Employing a milling technique, one can obtain a matrix of elongate bone particles containing at least 60 weight percent, preferably at least 70 weight percent, and most preferably at least 80 weight percent of elongate bone particles possessing a median length of greater than 1 mm to greater than 200 mm and preferably from 10 to 100 mm, and most preferably from 15 mm to 50 mm, a median thickness of from 0.05 to 2 mm, and preferably from 0.2 to 1 mm and a median width of from 1 mm to 20 mm, and preferably from 2 to 5 mm. These elongate bone particles can possess a median length to median thickness ratio of at least 5:1 up to 500:1 or more, and preferably from 50:1 to 100:1, and a median length to median width ratio of from 10:1 and 200:1, and preferably from 50:1 to 100:1. Another procedure for obtaining elongate bone particles, particularly useful for pieces of bone of up to 100 mm in length, is the bone processing mill described in commonly assigned U.S. Patent No. 5,607,269. Use of this bone mill results in the production of long, thin strips which quickly curl lengthwise to provide tubular-like bone particles. If desired, elongate bone particles can be graded into different sizes (e.g. by sieving) to reduce or eliminate any less desirable size(s) of particles which may be present In overall appearance, elongate bone particles can be described as filaments, fibers, threads, slender or narrow strips, etc.

At least 50 weight percent, more preferably at least 60 weight percent, and most preferably at least 90 weight percent of the bone particles present in the matrix of the osteoimplant herein are elongate. The balance of the bone particles can possess a wide range of dimensions, e.g., powders, chips, etc. The elongate bone particles form a coherent three-dimensional matrix which imparts cohesion, porosity and absorbency to the osteoimplant.

The bone particles utilized in the fabrication of the osteoimplant herein are demineralized in accordance with known and conventional procedures in order to reduce their inorganic mineral content. Demineralization methods remove the inorganic mineral component of bone by employing acid solutions. Such methods are well known in the art, see for example, Reddi et al., *Proc. Nat. Acad. Sci.* 69, pp 1601-1605 (1972). The strength of the acid solution, the shape of the bone particles and the duration of the demineralization treatment will determine the extent of demineralization. Reference in this regard may be made to Lewandrowski et al., *J. Biomed Materials Res,* 31, pp 365-372 (1996).

In a preferred demineralization procedure, the bone particles are subjected to an acid demineralization step followed by a defatting/disinfecting step. The bone particles are immersed in acid over time to effect their demineralization. Acids which can be employed in this step include inorganic acids such as hydrochloric acid and organic acids such as peracetic acid.

A preferred defatting/disinfectant solution is an aqueous solution of ethanol, the ethanol being a good solvent for lipids and the water being a good hydrophilic carrier to enable the solution to penetrate more deeply into the bone particles. The aqueous ethanol solution also disinfects the bone by killing vegetative microorganisms and viruses. Ordinarily, at least 10 to 40 percent by weight of water (i.e., 60 to 90 weight percent of defatting agent such as alcohol) should be present in the defatting disinfecting solution to produce optimal lipid removal and disinfection within the shortest period of time. The preferred concentration range of the defatting solution is from 60 to 85 weight percent alcohol and most preferably 70 weight percent alcohol. The treated demineralized bone particles are rinsed with sterile water to remove residual amounts of acid and thereby raise the pH. The wet demineralized elongate bone particles can then be stored under aseptic conditions, advantageously in a lyophilized state, for processing at a later time. As an alternative to aseptic processing and storage, the particles can be sterilized using known methods, e.g., gamma irradiation.

As utilized herein, the phrase "superficially demineralized" as applied to the bone particles refers to bone particles possessing at least about 90 weight percent of their original inorganic mineral content. The phrase "partially demineralized" as applied to the bone particles refers to bone particles possessing from 8 to 90 weight percent of their original inorganic mineral content, and the phrase "fully demineralized" as applied to the bone particles refers to bone particles possessing less than 8, preferably less than 1, weight percent of their original inorganic mineral content. The unmodified term "demineralized" as applied to the bone particles is intended to cover any one or combination of the foregoing types of demineralized bone particles.

Superficial or partial demineralization produces particles containing a mineralized core. Particles of this type increase the density and rigidity of the osteoimplant, through their mineralized core. These particles also play a biological role in bringing about new bone ingrowth by osteoinduction. Full demineralization produces particles in which nearly all of the mineral content has been removed from the particles. Particles treated in this way also contribute to the osteoinductivity of the osteoimplant. Nondemineralized bone particles act as a stiffener, providing density and rigidity to the osteoimplant Non-demineralized bone particles also play a biological role in bringing about new bone ingrowth by the process of osteoconduction. Thus, these bone particles are gradually remodeled and replaced by new host bone as incorporation of the osteoimplant progresses over time.

When prepared from bone particles that are almost exclusively fully and/or partially demineralized, the osteoimplant of the invention will be flexible and elastic. When particles that are nondemineralized and/or superficially demineralized are utilized in combination with fully and/or partially demineralized bone particles, the osteoimplant will increase in stiffness and rigidity. Thus, the use of combinations of different bone particles can be used to produce osteoimplants possessing properties, i.e., density, rigidity, osteoconductivity and/or osteoinductivity, etc. that are tailored to specific applications. The amount of each individual type of bone particle employed can vary widely depending on the mechanical and biological properties desired. Generally, the volume ratio of non demineralized and/or superficially demineralized bone particles to partially and/or fully demineralized bone particles can broadly range from 0:100 to 40:60. Suitable amounts can be readily determined by those skilled in the art on a case-by-case basis by routine experimentation.

If desired, the bone particles can be modified in one or more ways, e.g., their protein content can be augmented or modified as described in U.S. Patent Nos. 4,743,259 and 4,902,296.

The demineralized elongate bone particles are then combined with a wetting agent described hereinbelow to produce a composition containing from 5 to 40, preferably from 10 to 25, volume percent elongate demineralized bone particles, the remainder of the volume of the composition comprising wetting agent optionally in combination with one or more biocompatible components such as biocompatible binders, fillers, fibers, plasticizers, biostatic/biocidal agents, surface active agents, bioactive agents, and the like (further described hereinbelow). The wetting agent will cause the demineralized elongate bone particles to swell and increase in flexibility. The composition win possess a consistency ranging from a slurry or paste to a wet dough, depending on the amount of wetting agent used. The critical aspect is that the elongate bone particles be suspended in and evenly distributed throughout the wetting agent This is to be contrasted with the "wet laying" procedure of commonly assigned U.S. Patent No. 5,507,813, in which wetting agent is substantially removed to produce a dense mat of bone particles.

The composition is typically formed by mixing bone particles and wetting agent to form a liquid slurry, stirring the slurry for a suitable period of time sufficient to allow the wetting agent to penetrate the demineralized elongate bone particles, and removing enough wetting agent, e.g., by draining through a sieve, sufficient to provide a composition containing from 5 to 40, preferably from 10 to 25, volume percent bone particles.

Suitable wetting agents typically comprise water and may optionally further include biocompatible liquids such as organic protic solvent, aqueous solution such as physiological saline, concentrated saline solutions, sugar solutions, ionic solutions of any kind, and liquid polyhydroxy compounds such as glycerol and glycerol esters, polyoxyalkylenes (e.g., Pluronics®), and mixtures thereof.

Optionally, the wetting agent can comprise dissolved or admixed therein one or more biocompatible substances such as biocompatible binders, fillers, plasticizers, biostatic/biocidal agents, surface active agents, bioactive substances, etc, as disclosed in commonly-assigned published International Application WO 00/50102.

Suitable binders include cyanoacrylates, epoxy-based compounds, dental resin sealants, dental resin cements, calcium phosphate and calcium sulfate self-setting cements, glass ionomer cements, polymethyl methacrylate, gelatin-resorcinol-formaldehyde glues, protein and collagen-based glues, acrylic resins, cellulosics, bioabsorbable polymers such as polyglycolide, polylactide, glycolide-lactide copolymers, polycaprolactone, polyanhydrides, polycarbonates, polyorthoesters, polyamino acids, polyarylates, polycyanoacrylates, polyhydroxybutyrate, polyhydroxyvalyrate, polyphosphazenes, and polyvinylpyrrolidone, etc.

Suitable fillers include bone powder, demineralized bone powder, porous calcium phosphate ceramics; hydroxyapatite, tricalcium phosphate, Bioglass® and other calcium phosphate materials, calcium sulfate or calcium carbonate particles, etc.

Suitable plasticizers include liquid polyhydroxy compounds such as glycerol, monoacetin, diacetin, hydrogels, etc.

Suitable biostatic/biocidal agents include antibiotics, povidone, sugars, mucopolysaccharides, etc.

Suitable surface active agents include the biocompatible nonionic, cationic, anionic and amphoteric surfactants.

It will be understood by those skilled in the art that the foregoing list is not intended to be exhaustive and that other materials may be employed such as those disclosed in U.S. Patent No. 5,073,373.

Bioactive substances include physiologically or pharmacologically active substances that act locally or systemically in the host. Representative classes of bioactive factors which can be readily combined with the bone particles include, e.g., trophic factors, analgesics, anticancer agents, vaccines, adjuvants, antibodies, neuroleptics, genes and genetic elements for transfection, cells or cellular components, etc. A list of more specific examples would therefore include, collagen, insoluble collagen derivatives, etc., and soluble solids and/or liquids dissolved therein, e.g., antiviricides, particularly those effective against HIV and hepatitis; antimicrobials and/or antibiotics such as erythromycin, bacitracin, neomycin, penicillin, polymicin B, tetracyclines, biomycin, chloromycetin, and streptomycins, cephalosporins, ampicillin, azactam, tobramycin, clindamycin and gentamicin, etc.; biocidal/biostatic sugars such as dextran, glucose, etc.; amino acids, peptides, vitamins, inorganic elements, co-factors for protein synthesis; hormones; endocrine tissue or tissue fragments, synthesizers; enzymes such as collagenase, peptidases, oxidases, etc., polymer cell scaffolds with parenchymal cells, angiogenic drugs and polymeric carriers containing such drugs; collagen lattices; antigenic agents; cytoskeletal agents; cartilage fragments, modified living cells such as chondrocytes, bone marrow cells, mesenchymal stem cells, natural extracts, genetically engineered living cells or otherwise modified living cells, DNA delivered by plasmid or viral vectors, genes or genetic elements, tissue transplants, demineralized bone powder, autogenous tissues such as blood, serum, soft tissue, bone marrow, etc.; bioadhesives; non-collagenous proteins such as osteopontin, osteonectin, bone sialo protein, laminin, fibrinogen, vitronectin, thrombospondin, proteoglycans, decorin, beta glycan, biglycan, aggrecan, versican, tenascin, matrix gla protein, hyaluronan, amino acids, amino acid residues, peptides, bone morphogenic proteins (BMPs); osteoinductive factor (OIF); fibronectin (FN); endothelial cell growth factor (ECGF); cementum attachment extracts (CAE); ketanserin; human growth hormone (HGH); animal growth hormones; epidermal growth factor (EGF); interleukin-1 (IL-1); human alpha thrombin; transforming growth factor (TGF-beta); insulin-like growth factor (IGF-1) (IGF-2); platelet derived growth factors (PDGF); fibroblast growth factors (FGF, bFGF, etc.); periodontal ligament chemotactic factor (PDLGF); somatotropin; bone digestors; antitumor agents; immunosuppressants; permeation enhancers, e.g., fatty acid esters such as laureate, myristate and stearate monoesters of polyethylene glycol, enamine derivatives, alpha-keto-aldehydes, etc.; and nucleic acids; inorganic elements, inorganic compounds, cofactors for protein synthesis, hormones, soluble and insoluble components of the immune system; soluble and insoluble receptors including truncated forms; soluble, insoluble and cell surface bound ligands including truncated forms; chemokines, bioactive compounds that are endocytosed; endocrine tissue or tissue fragments, growth factor binding proteins, e.g., insulin-like growth factor binding protein (IGFBP-2) (IGFBP-4) (IGFBP-5) (IGFBP-6); angiogenic agents, bone promoters, cytokines, interleukins, genetic material, genes encoding bone promoting actions, cells containing genes encoding bone promoting action; growth hormones such as somatotrophin; bone digestors, antiumor agents; cellular attractants and attachment agents; immunosuppressants; bone resorption inhibitors and stimulators; angiogenic and mitogenic factors; bioactive factors that inhibit and stimulate secondary messenger molecules; cell adhesion molecules, e.g., cell-matrix and cell-cell adhesion molecules; secondary messengers, monoclonal antibodies specific to cell surface determinants on mesenchymal stem cells, clotting factors; externally expanded autograft or xenograft cells, nucleic acids and any combination thereof. The amounts and types of such optionally added substances can vary widely with optimum levels and combinations being readily determined in a specific case by routine experimentation.

Preferred wetting agents for forming the wetted matrix of bone particles include mixtures/solutions of water and liquid polyhydroxy compounds and their esters, and and/or surface active agents. The preferred polyhydroxy compounds possess up to about 12 carbon atoms and, where their esters are concerned, are preferably the monoesters and diesters. Specific polyhydroxy compounds of the foregoing type include glycerol and its monoesters and diesters derived from low molecular weight carboxylic acids, e.g., monoacetin and diacetin (respectively, glycerol monoacetate and glycerol diacetate), ethylene glycol, diethylene glycol, triethylene glycol, 1,2-propanediol, trimethylolethane, trimethylolpropane, pentaerythritol, sorbitol, polyoxyalkylenes, e.g., Pluronics®, and the like. Of these, glycerol is especially preferred as it improves the handling characteristics of the bone particles wetted therewith and is biocompatible and easily metabolized. Most preferred are glycerol/water solutions in weight ratios ranging from 40:60 to 5:95, respectively. Mixtures of polyhydroxy compounds or esters, e.g., sorbitol dissolved in glycerol, glycerol combined with monoacetin and/or diacetin, etc., are also useful.

Where, in a particular composition, the bone particles have a tendency to quickly or prematurely separate or to otherwise settle out from the wetted matrix such that application of a fairly homogeneous composition is rendered difficult or inconvenient, it can be advantageous to include within the composition a substance whose thixotropic characteristics prevent or reduce this tendency. Thus, e.g., where the wetting agent is water and/or glycerol and separation of bone particles occurs to an excessive extent where a particular application is concerned, a thickener such as a solution of polyvinyl alcohol, polyvinylpyrrolidone, cellulosic ester such as hydroxypropyl methylcellulose, carboxy methylcellulose, pectin, xanthan gum, food-grade texturizing agent, gelatin, dextran, collagen, starch, hydrolyzed polyacrylonitrile, hydrolyzed polyacrylamide, polyelectrolyte such as polyacrylic acid salt, hydrogels, chitosan, other materials that can suspend particles, etc., can be combined with the wetting agent in an amount sufficient to significantly improve the suspension-keeping characteristics of the composition.

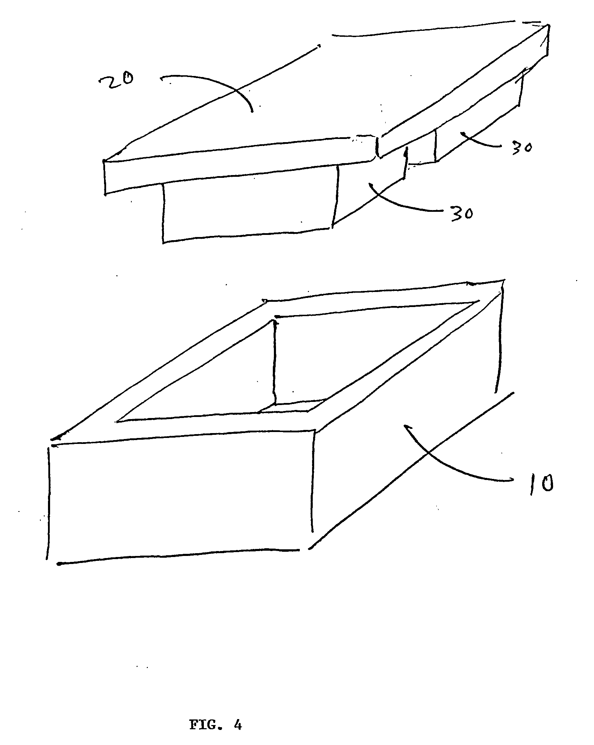

The composition is next placed in a mold possessing any desired shape or configuration. Fig. 4 depicts mold 10 and lid 20 for mold 10, lid 20 possessing protruding indentations 30. The mold can be optionally configured and dimensioned in the shape of the final osteoimplant. Care must be taken to ensure that minimal, if any, pressure is applied to the composition which would effect compaction of the bone particles. This is in contradistinction to the wet-lay procedure described in U.S. Patent No. 5,507,813. The composition is heated in the mold at a temperature above 35°C, preferably from 35°C to 70 °C, more preferably from 40°C to 50°C for a suitable period of time, e.g., from 3 to 4 hours, to effect removal of water. The resulting material comprises a shaped, coherent, three-dimensional porous matrix of elongate demineralized bone particles, wherein said matrix possesses a bulk density of less than 0.3.g/cm³. The bulk density of the implant will typically range from 0.01 to 0.3, preferably from 0.05 to 0.2, g/cm³. Following the heating step, the shaped material is lyophilized, e.g., using a shelf temperature of from -20° to -70°C, a vacuum of from 150 to 100 mTorr at a time of from 4 to 48 hours. Lyophilization improves the long term stability of the implant and eliminates the need for special preservation steps such as freezing or the use of preservatives. The resulting lyophilized material will comprise less than 5 weight percent water and is porous, absorbent, does not require hydration for pliability and clinical use, and maintains its shape and cohesiveness upon absorption of fluid.

Optionally, the bone particles can be cross-linked in accordance with well known techniques, e.g., those disclosed in the aforementioned International Application WO 00/50102.

In accordance with a preferred embodiment, the osteoimplant of the invention is combined with a flowable osteogenic material such as autologous bone graft, bone marrow aspirate, demineralized bone matrix (DBM), bone morphogenic protein (BMP), and the like. In a preferred embodiment, the osteoimplant is provided with one or more cavities or depressions which can be filled with the flowable osteogenic material. The cavities or depressions can be formed by employing a mold possessing a lid having indentations therein. Fig. 4 depicts mold 10 having lid 20 with protruding indentations 30 therein. Alternatively, the cavities or depressions can be formed by cutting the osteoimplant.

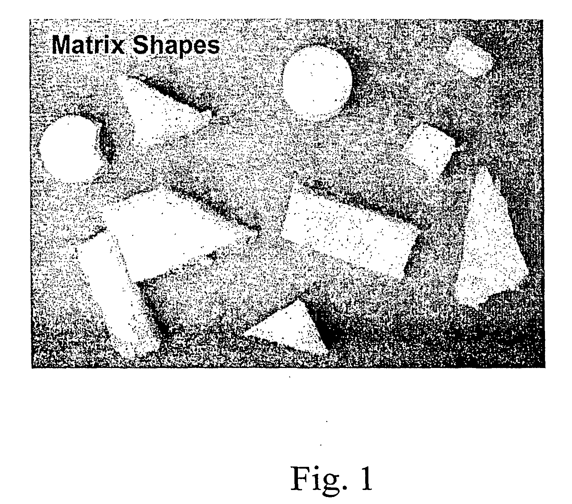

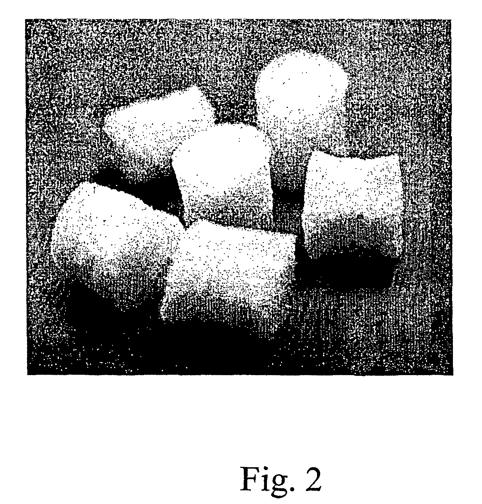

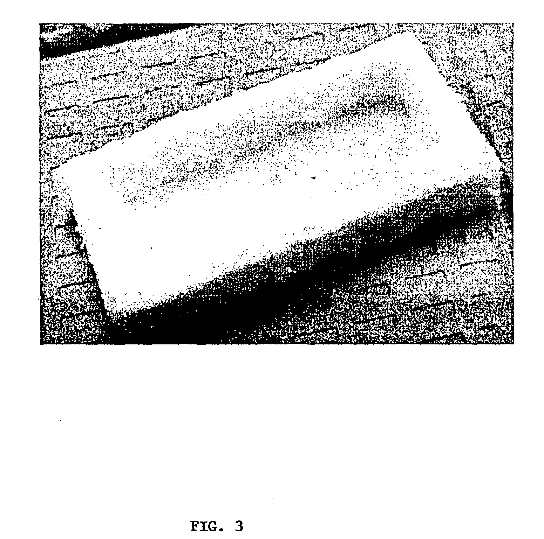

The osteoimplant can assume a determined or regular form or configuration such as a sheet, plate, disk, cone, pin, screw, tube, tooth, tooth root, bone or portion of bone, wedge or portion of wedge, cylinder, threaded cylinder (to name but a few). Reference can be made to the photographs of Fig. 1-3 which depict various useful embodiments of the invention. The osteoimplants of Figs. 1-3 were produced using the method described herein. It can be readily seen that the implants are porous and flexible in nature. The osteoimplant can be cut either in the dry state or in the wetted state. The osteoimplant can be utilized in a wide variety of orthopedic, periodontal, neurosurgical and oral and maxillofacial surgical procedures such as the repair of simple and compound fractures and non-unions, external and internal fixations, joint reconstructions such as arthrodesis, general arthroplasty, cup arthroplasty of the hip, femoral and humeral head replacement, femoral head surface replacement and total joint replacement, repairs of the vertebral column including spinal fusion and internal fixation, tumor surgery, e.g., deficit filling, discectomy, laminectomy, excision of spinal cord tumors, anterior cenical and thoracic operations, repairs of spinal injuries, scoliosis, lordosis and kyphosis treatments, intermaxillary fixation of fractures, mentoplasty, temporomandibular joint replacement, alveolar ridge augmentation and reconstruction onlay bone grafts, implant placement and revision, sinus lifts, etc. Specific bones which can be repaired or replaced with the osteoimplant herein include the ethmoid, frontal, nasal, occipital, parietal, temporal, mandible, maxilla, zygomatic, cervical vertebra, thoracic vertebra, lumbar vertebra, sacrum, rib, sternum, clavicle, scapula, humerus, radius, ulna, carpal bones, metacarpal bones, phalanges, ilium, ischium, pubis, femur, tibia, fibula, patella, calcaneus, tarsal and metatarsal bones. The osteoimplant can be implanted at the bone repair site, if desired, using any suitable affixation means, e.g., sutures, staples, bioadhesives, and the like. In accordance with one embodiment, the osteoimplant is configured and dimensioned as cylinders of approximately 5 mm diameter, 1 cm long that can be placed into tooth extraction sockets.

The following examples illustrate the practice of the present invention and in no way limit the scope of the claims appended hereto.

### EXAMPLE 1

### Process of making a species-specific osteoimplant with defined dimensions.

Species-specific (Rhesus Monkey) long bones were aseptically cleaned. The cortical bone was processed in the bone milling apparatus described in U.S. Patent No. 5,607,269 to yield 65 grams of elongate bone particles. The elongate bone particles were placed in a reactor and allowed to soak for 5 -10 minutes in 0.6N HCl plus 20-2000 ppm nonionic surfactant solution. Following drainage of the HCl/surfactant, 0.6N HCl at 15ml per gram of total bone was introduced into the reactor along with the elongate bone particles. The reaction proceeded for 40-50 minutes. Following drainage through a sieve, the resulting demineralized elongate bone particles were rinsed three times with sterile, deionized water at 15ml per gram of total bone, being replaced at 15 minute intervals. Following drainage of the water, the bone particles were covered in alcohol and allowed to soak for at least 30 minutes. The alcohol was then drained and the bone particles were rinsed with sterile deionized water. The bone particles were then contacted with a mixture of 4.5 ml glycerol per gram of dry bone particles and 10.5 ml sterile, deionized water per gram of dry bone particles for at least 60 minutes. Excess liquid was drained and the resulting liquid composition containing approximately 11 (w/v) demineralized elongate bone particles was transferred to a 11 cm X 11 cm mold containing a lid having a plurality of protruding indentations such as those depicted in Fig. 4. The dimensions of the protrusions were specific for the size of the osteoimplant required for the Rhesus monkey. The lid was gently placed on the mold such that the indentations became immersed into the liquid composition to exert as little pressure on the composition as possible. The mold was then placed in an oven at 46°C for 4 hours. The composition was then frozen overnight at -70°C and then lyophilized for 48 hours. Following lyophilization, the mold was disassembled and the formed composition was cut into individual pieces that contained troughs corresponding to the dimensions of the lid protrusions. The resulting pieces had dimensions of 4.5 cm in length, 2.5 cm in width and about 8 mm in height with trough dimensions of 3.5 cm in length, 1 cm in width and 4 mm of depth.

The resulting composition was cohesive, flexible, and sponge-like with an obvious continuous three-dimensional structure possessing visible open pores. The implant had a defined shape including the indentations made by the lid protrusions, did not require rehydration before use, and was more rapidly hydratable in comparison to Grafton®Flex. The material retained its shape once wetted with fluids and freezing was not required for storage.

The density of bone is based on calculation of the defined mold volume used and the amount of demineralized bone particles used to fill the volume of the mold. In making the composition described in this example, 12g demineralized fibers occupied a volume of 105cm³. Therefore, the density was approximately 0.114g of bone/cm³. These calculations are approximate as there can be a range in weights (about 10-20g) and a range in volumes of about 100-120cm³ (which can be defined by the dimensions of the mold used).

### EXAMPLE 2

### Evidence of Osteoinduction by Grafton DBM in Non-Human Primate Spine Fusion.

While autogenous iliac crest bone graft remains the "gold standard", much work continues to identify viable bone graft extenders, enhancers, and substitutes. While several demineralized bone matrix formulations have been shown to be variably osteoinductive in rodent ectopic bone assays, few have demonstrated efficacy in higher species and more challenging applications such as posterolateral spine fusion. To date, none have been tested in a non-human primate posterolateral spine fusion model which has been previously determined to be extremely challenging with less than 40% of animals achieving successful fusion with autogenous iliac crest bone graft. The purpose of this example was to test the osteoimplant described in Example 1 for evidence of osteoinduction and its use as an extender/enhancer for autogenous bone graft in a non-human primate.

Four skeletally mature rhesus macaques underwent single level lumbar posterolateral arthrodesis through a Wiltse muscle-splitting approach under general anesthesia. The transverse processes were decorticated with an electric burr. Autogenous iliac crest bone graft was harvested bilaterally through separate fascial incisions. In these four animals, rhesus-specific osteoimplant material (described in Example 1) was implanted with the usual autograft (4g) on one side of the spine and one half the usual autograft (2g) on the opposite side. Radiographs were taken at intervals until euthanasia at 24 weeks. The lumbar spines were excised and palpated manually to determine fusion status as fused or not fused and then underwent CT scanning to visualize the amount of bone formation. Radiographs and CT scans were evaluated blindly and assessed semi-quantitatively for the area of the fusion mass (3=good, 2=fair, 1=poor) and the amount of bridging between the transverse processes on each side (0=<25%,1=25%, 2=50%, 3=75%,4=100%). Points were added for each site in each animal. Three of four monkeys receiving the osteoimplant plus autograft were graded as fused. Six of eight sites in the were rated as "good" for area of fusion mass on CT (computer tomography) scans. Six of eight sites had at least 50% bridging. The quality and amount of bone was better in the osteoimplant group and best with the 4g of autograft. Although the assessment of bone formation was semi-quantitative, given the spectrum of fusions previously obtained in this model with autograft alone, these data support evidence of osteoinduction of the osteoimplant in a challenging model. These data support the role of this osteoimplant as an osteoinductive graft extender and graft enhancer in rhesus posterolateral spine fusion.

### EXAMPLE 3

### Implantation of Osteoimplant in a human patient to promote spinal fusion.

Human-specific osteoimplant was made in the same manner described in Example 1. However, the mold dimensions and final dimensions of the osteoimplant were altered to adjust to the approximate size required for human posterolateral spinal fusion procedure (known by those skilled in the art). The dimensions of the osteoimplant pieces were approximately 5.0cm in length, 2.5cm in width and approximately 1cm in height with trough dimensions 4cm in length, 1.5cm in width and depth approximately 0.7cm. The trough design specifically allowed for the surgeon to fill the center of the osteoimplant with autograft or allograft or both. Autograft is usually obtained from local bone at the site of the procedure, or marrow, or iliac crest or a combination. The fluids rapidly dispersed within the osteoimplant hydrating the osteoimplant. The osteoimplant is placed either trough down facing the decorticated transverse processes or trough facing away from the decorticated transverse processes to allow blood to be absorbed by the sponge-like nature of the osteoimplant The osteoimplnat remains as a three-dimensional cohesive structure retaining the autograft or allograft or both at the implant site. The surgery then follows usual closure procedure known to those skilled in the art.

### EXAMPLE 4

### Evaluation of the Osteoinductive Potential of Example 1.

The osteoinductive potential of Example 3 (human-specific osteoimplant) for posterolateral fusion (PLF) was evaluated using the standard heterotopic osteoinductive implant model (see, Edwards JT, Diegmann MH, Scarborough NL, Osteoinduction of human demineralized bone: Characterization in an animal model, Clin Orthop ReI Res 357:219228 (1998) which is a modification ofUrist MR, Bone formation by autoinduction. Science, 150:893-899 (1965)) . Implants are placed in the hind limb, intramuscular sites of athymic rats and evaluated histologically after 28 days.

### Animal Model

The study was conducted in the athymic (nude) rat to minimize the potential for a cross species incompatibility response to xenograft tissue implants. The hind-limb intramuscular site is ideal for the initial determination of heterotopic bone induction properties of implant materials, as bone is not present in this area.

### Implant Placement

The study utilized a singular intramuscular (IM) implantation site in each hind limb of the animals. Different speciment types were placed in the sites in a randomized fashion, such that the same animal did not have the same treatment in both hind limbs. To provide a common positive control over all animals, a single 40mg sample of rat DBM powder was placed intramuscularly over the left pectoralis (LP) muscle on the left side of each rat. Animals were allowed normal activities following surgical procedures. Four samples of each material were used for analysis.

### Procedure

Briefly, rats were anesthetized with a mixture of ketamine (250mg), xylazine (11mg), and physiological saline (10ml). The dosage is 3.6ml/kg body weight administered intraperitoneally. Aseptic surgical procedures were carried out in a laminar airflow hood. A 1cm skin incision was made on each upper hind limb using a lateral approach and the skin was separated from the muscle by blunt dissection. A superficial incision aligned with the muscle fiber plane was made to allow for insertion of the tips of the scissors. Blunt dissection of the muscle to create a pocket and positioning of the rat DBM powder or devitalized fibers was made using a blunt syringe. In each case, the skin was closed with metal clips.

Rats were euthanized with CO₂ following 28-day implantation time. Implant materials were located by palpitation, retrieved by blunt dissection and cleaned of the surrounding tissue by careful trimming. An observer blinded to implant type performed a macroscopic evaluation of the implant material. Color, vascularity, hardness and integrity were scored according to the scheme outlined in Table I; the highest score for the most robust response would be 1, while a specimen showing little or no osteoinductive potential would score 0. Experience with this model has shown a high correlation between visual observations and histological observations of DBM implant performance.

### Histology

Retrieved materials were fixed in neutral buffered formalin, dehydrated in a series of graded ethanol solutions, embedded in JB-4 (glycol methacrylate, Polysciences, Inc., Warrington, PA) and sectioned. Toluidine blue was used for staining and each material was evaluated using a light microscope at magnifications up to 200X.

A numerical score of 0, 1, 2, 3, or 4 was given to grade the extent of new bone formation for each explant when examined under the light microscope. Assignment of scores was according to the descriptions given in Table II below. Histological sections for each explant were scored independently by two individuals blinded to treatment groups.

Following histological analysis, average scores were calculated for each material type or sample group. Based on previous experience with this animal model, each group was assigned an assessment of osteoinductive potential based on the average histological scores. Sample groups scoring 0 show "no osteoinductive response"; groups scoring up to 2 show a "slight osteoinductive response" and groups scoring 3 or above show a "robust osteoinductive response".

**Table I**

| Macroscopic Observation Scoring Guidelines | | | |
|---|---|---|---|
| Color | White (W) | Gray (G) | Red(R) |
| Vascularity | None (N) | Some (S) | Robust (R) |
| Hardness | Mushy (M) | Firm (F) | Hard(H) |
| Integrity | Diffuse (D) | Flat (F) | Nodule (N) |
| Score | 0 | 0.5 | 1 |

**Table II**

| Scoring of Histological Sections | |
|---|---|
| Score | New Bone Formation |
| 0 | No new bone |
| 1 | Few areas of new bone formation |
| 2 | Numerous areas of new bone formation |
| 3 | Greater than 50% of nodule involved in new bone formation |
| 4 | Greater than 75% of nodule involved in new bone formation |

### Results

Histology showed evidence of robust cartilage, bone and marrow formation in the samples. Scores for the individual samples were averaged and the mean ± SD of the osteoinductive score for 13 individual samples derived from Example 3 was 3.3 ± 0.7. Historically, demineralized bone powder produces a comparable osteoinductive score of 3.6 ± 0.8 while guanidine hydrochloride extracted samples routinely display lack of inductivity. The foregoing results demonstrate that the osteoimplant of the invention possesses excellent osteoinductivity with the additional advantage of being a cohesive three-dimensional, lower density, porous matrix.

## Claims

1. An osteoimplant which comprises a shaped, coherent, three-dimensional porous matrix of elongate demineralized bone particles, wherein said matrix possesses a bulk density of lower than 0.3 g/cm³.

2. The osteoimplant of claim 1 wherein the matrix further contains one or more biocompatible components.

3. The osteoimplant of claim 2 wherein the biocompatible components are selected from the group consisting of biocompatible binder, filler, fiber, plasticizer, biostatic/biocidal agent, surface active agent, and bioactive substance.

4. The osteoimplant of any one of claims 1 to 3 wherein the elongate bone particles represent at least 50 weight percent of the matrix.

5. The osteoimplant of claim 4 wherein the elongate bone particles represent at least 60 weight percent of the matrix.

6. The osteoimplant of claim 5 wherein the elongate bone particles represent at least 90 weight percent of the matrix.

7. The osteoimplant of anyone of claims 1 to 6 wherein the matrix further comprises bone particles possessing dimensions other than elongate.

8. The osteoimplant of anyone of claims 1 to 7 wherein the matrix further comprises nondemineralized bone particles.

9. The osteoimplant of anyone of claims 1 to 8 wherein the elongate demineralized bone particles are selected from the group consisting of fully, partially and superficially demineralized bone particles, and combinations thereof.

10. The osteoimplant of anyone of claims 1 to 9 wherein the elongate demineralized bone particles possess a median length to median thickness ratio of at least 5:1 up to 500:1.

11. The osteoimplant of anyone of claims 1 to 10 wherein the matrix comprises less than 5 weight percent water and is flexible.

12. The osteoimplant of anyone of claims 1 to 11 wherein the matrix comprises less than 5 weight percent water and possesses an osteoinductivity of at least 2 when measured in an athymic rat assay.

13. The osteoimplant of anyone of claims 1 to 12 wherein the elongate demineralized bone particles are cross-linked.

14. The osteoimplant of anyone of claims 1 to 13 wherein the bulk density of the osteoimplant ranges from 0.01 to 0.3 g/cm³.

15. The osteoimplant of anyone of claims 1 to 14 wherein the bulk density of the osteoimplant ranges from 0.05 to 0.2 g/cm³.

16. The osteoimplant of anyone of claims 1 to 15 in the shape of a sheet, plate, disk, cone, pin, screw, tube, tooth, tooth root, bone or portion of bone, wedge or portion of wedge, cylinder, and threaded cylinder.

17. The osteoimplant of anyone of claims 1 to 16, comprising at least one cavity or depression.

18. A method of fabricating an osteoimplant which comprises:
a) providing a quantity of elongate demineralized bone particles;
b) mixing the elongate demineralized bone particles with a wetting agent comprising water to provide a liquid composition containing from 5 to 40 volume percent swollen, hydrated elongate demineralized bone particles;
c) placing the liquid composition in a mold; and,
d) heating the liquid composition in the substantial absence of pressure at temperature above 35° C for a period of time sufficient to remove water present in the wetting agent to provide an osteoimplant comprising a shaped, coherent, three-dimensional porous matrix of elongate demineralized bone particles, wherein said matrix possesses a bulk density of lower than 0.3 g/cm³.

19. The method of claim 18 further comprising incorporating one or more biocompatible components in the liquid composition.

20. The method of claim 19 wherein the biocompatible components are selected from the group consisting of biocompatible binder, filler, fiber, plasticizer, biostatic/biocidal agent, surface active agent, and bioactive substance.

21. The method of anyone of claims 18 to 20 wherein the elongate demineralized bone particles represent at least 50 weight percent of the matrix.

22. The method of anyone of claims 18 to 21 wherein the elongate demineralized bone particles represent at least 60 weight percent of the matrix.

23. The method of anyone of claims 18 to 22 wherein the elongate demineralized bone particles represent at least 90 weight percent of the matrix.

24. The method of anyone of claims 19 to 23 wherein the biocompatible component comprises bone particles possessing dimensions other than elongate.

25. The method of anyone of claims 19 to 24 wherein the biocompatible component comprises nondemineralized bone particles.

26. The method of anyone of claims 18 to 25 wherein the elongate demineralized bone particles are selected from the group consisting of fully, partially and superficially demineralized bone particles, and combinations thereof.

27. The method of anyone of claims 18 to 26 wherein the elongate demineralized bone particles possess a median length to median thickness ratio of at least 5:1 up to 500:1.

28. The method of anyone of claims 18 to 27 wherein the wetting agent comprises water in combination with one or more components selected from the group consisting of organic protic solvent, physiological saline, concentrated saline solutions, sugar solutions, ionic solutions, liquid polyhydroxy compounds, and polyoxyalkylene compounds.

29. The method of anyone of claims 18 to 28 wherein the wetting agent comprises water and polyhydroxy compound.

30. The method of claim 29 wherein the polyhydroxy compound is glycerol.

31. The method of claim 30 wherein the weight ratio of glycerol to water ranges from 40:60 to 5:95.

32. The method of anyone of claims 18 to 31 wherein after heating step d, the osteoimplant is subjected to a further treatment step.

33. The method of claim 32 wherein the further treatment step is selected from the group consisting of lyophilizing, crosslinking, re-mineralization, sterilization, machining, laser etching, welding, assembling of parts, cutting, milling and reactive etching.

34. The method of claim 32 wherein the further treatment step is lyophilizing.

35. The osteoimplant of anyone of claims 1 to 34 comprising at least one cavity or depression.

36. The osteoimplant of claim 35 wherein the cavity or depression contains a flowable osteogenic material.

37. The osteoimplant of claim 36 wherein the flowable osteogenic material is selected from the group consisting of autologous bone graft, bone marrow aspirate, demineralized bone matrix and bone morphogenic protein.

38. Use of an osteoimplant according to anyone of claims 1 to 37 for the manufacture of an osteoimplant which comprises a shaped, coherent, three-dimensional porous matrix of elongate demineralized bone particles, wherein said matrix possesses a bulk density of lower than 0.3 g/cm³.

39. Use according to claim 38 wherein the repaired bone is selected from the group consisting of the ethmoid, frontal, nasal, occipital, parietal, temporal, mandible, mayilla, zygomatic, cervical vertebra, thoracic vertebra, lumar vertebra, scarum, rib, sternum, clavicle, scapula, humerus, radius, ulna, carpal bones, metacarpal bones phalanges, ilium ischium, pubis, femur, tibia, fibula, patella, calcaneus, tarsal and metatarsal bones.

40. Use of an osteoimplant according to anyone of claims 1 to 37 for disposing said osteoimplant between adjacent vertebrae of the spine.

41. Use according to claim 40 wherein the osteoimplant comprises at least one cavity or depression which contains at least one bone-growth inducing substance therein.

## Patentansprüche

1. Osteoimplant, enthaltend eine geformte, kohärente dreidimensionale poröse Matrix aus länglichen demineralisierten Knochenpartikeln, **dadurch gekennzeichnet, dass** die Matrix eine Schüttdichte von weniger als 0,3 g/cm³ hat.

2. Osteoimplant nach Anspruch 1, **dadurch gekennzeichnet, dass** die Matrix ein oder mehrere biokompatible Komponenten enthält.

3. Osteoimplant nach Anspruch 2, **dadurch gekennzeichnet, dass** die biokompatiblen Komponenten ausgewählt sind aus der aus biokompatiblen Bindemitteln, Füllstoffen, Fasern, Weichmachern, biostatischen/bioziden Mitteln, Tensiden und biologisch aktiven Substanzen bestehenden Gruppe.

4. Osteoimplant nach einem der Ansprüche 1 bis 3, **dadurch gekennzeichnet, dass** die länglichen Knochenpartikel wenigstens 50 Gew.-Prozent der Matrix ausmachen.

5. Osteoimplant nach Anspruch 4, **dadurch gekennzeichnet, dass** die länglichen Knochenpartikel wenigstens 60 Gew.-Prozent der Matrix ausmachen.

6. Osteoimplant nach Anspruch 5, **dadurch gekennzeichnet, dass** die länglichen Knochenpartikel wenigstens 90 Gew.-Prozent der Matrix ausmachen.

7. Osteoimplant nach einem der Ansprüche 1 bis 6, **dadurch gekennzeichnet, dass** die Matrix weiterhin Knochenpartikel enthält, die Dimensionen aufweisen, die nicht länglich sind.

8. Osteoimplant nach einem der Ansprüche 1 bis 7, **dadurch gekennzeichnet, dass** die Matrix weiterhin nicht-demineralisierte Knochenpartikel enthält.

9. Osteoimplant nach einem der Ansprüche 1 bis 8, **dadurch gekennzeichnet, dass** die länglichen demineralisierten Knochenpartikel aus der aus vollständig, teilweise und oberflächlich demineralisierten Knochenpartikeln und Kombinationen davon bestehenden Gruppe ausgewählt sind.

10. Osteoimplant nach einem der Ansprüche 1 bis 9, **dadurch gekennzeichnet, dass** die länglichen demineralisierten Knochenpartikel ein Verhältnis von medianer Länge zu medianer Dicke von wenigstens 5:1 bis zu 500:1 aufweisen.

11. Osteoimplant nach einem der Ansprüche 1 bis 10, **dadurch gekennzeichnet, dass** die Matrix weniger als 5 Gew.-Prozent Wasser enthält und flexibel ist.

12. Osteoimplant nach einem der Ansprüche 1 bis 11, **dadurch gekennzeichnet, dass** die Matrix weniger als 5 Gew.-Prozent Wasser enthält und eine im Assay mit athymischen Ratten gemessene Osteoinduktivität von wenigstens 2 aufweist.

13. Osteoimplant nach einem der Ansprüche 1 bis 12, **dadurch gekennzeichnet, dass** die länglichen demineralisierten Knochenpartikel quervernetzt sind.

14. Osteoimplant nach einem der Ansprüche 1 bis 13, **dadurch gekennzeichnet, dass** die Schüttdichte des Osteoimplants im Bereich von 0,01 bis 0,3 g/cm³ liegt.

15. Osteoimplant nach einem der Ansprüche 1 bis 14, **dadurch gekennzeichnet, dass** die Schüttdichte des Osteoimplants im Bereich von 0,05 bis 0,2 g/cm³ liegt.

16. Osteoimplant nach einem der Ansprüche 1 bis 15 in Form eines Blatts, einer Platte, einer Scheibe, eines Kegels, einer Nadel, einer Schraube, eines Röhrchens, eines Zahns, einer Zahnwurzel, eines Knochens oder Knochenteils, eines Keils oder eines Teils eines Keils, eines Zylinders und eines mit einem Gewinde versehenen Zylinders.

17. Osteoimplant nach einem der Ansprüche 1 bis 16, enthaltend wenigstens einen Hohlraum oder eine Vertiefung.

18. Verfahren zur Herstellung eines Osteoimplants, bei dem man:
a) längliche demineralisierte Knochenpartikel bereitstellt;
b) die länglichen demineralisierten Knochenpartikel mit einem wasserhaltigen Netzmittel mischt und so eine flüssige Zusammensetzung bereitstellt, die 5 bis 40 Vol.-Prozent gequollene hydratisierte längliche demineralisierte Knochenpartikel bereitstellt;
c) die flüssige Zusammensetzung in eine Form gibt; und
d) die flüssige Zusammensetzung so lange im wesentlichen drucklos auf eine Temperatur über 35°C erhitzt, bis das im Netzmittel vorhandene Wasser entfernt ist, wodurch ein Osteoimplant bereitgestellt wird, das eine geformte, kohärente dreidimensionale poröse Matrix aus länglichen demineralisierten Knochenpartikeln enthält, die **dadurch gekennzeichnet ist, dass** die Matrix eine Schüttdichte von weniger als 0,3 g/cm³ hat.

19. Verfahren nach Anspruch 18, bei dem man in die flüssige Zusammensetzung weiterhin eine oder mehrere biologisch kompatible Komponenten einarbeitet.

20. Verfahren nach Anspruch 19, **dadurch gekennzeichnet, dass** die biokompatiblen Komponenten ausgewählt sind aus der aus biokompatiblen Bindemitteln, Füllstoffen, Fasern, Weichmachern, biostatischen/bioziden Mitteln, Tensiden und biologisch aktiven Substanzen bestehenden Gruppe

21. Verfahren nach einem der Ansprüche 18 bis 20, **dadurch gekennzeichnet, dass** die länglichen demineralisierten Knochenpartikel wenigstens 50 Gew.-Prozent der Matrix ausmachen.

22. Verfahren nach einem der Ansprüche 18 bis 21, **dadurch gekennzeichnet, dass** die länglichen demineralisierten Knochenpartikel wenigstens 60 Gew.-Prozent der Matrix ausmachen.

23. Verfahren nach einem der Ansprüche 18 bis 22, **dadurch gekennzeichnet, dass** die länglichen demineralisierten Knochenpartikel wenigstens 90 Gew.-Prozent der Matrix ausmachen.

24. Verfahren nach einem der Ansprüche 19 bis 23, **dadurch gekennzeichnet, dass** die biologisch kompatiblen Komponenten weiterhin Knochenpartikel enthalten, die Dimensionen aufweisen, die nicht länglich sind.

25. Verfahren nach einem der Ansprüche 19 bis 24, **dadurch gekennzeichnet, dass** die biologisch kompatiblen Komponenten weiterhin nicht-demineralisierte Knochenpartikel enthalten.

26. Verfahren nach einem der Ansprüche 18 bis 25, **dadurch gekennzeichnet, dass** die länglichen demineralisierten Knochenpartikel aus der aus vollständig, teilweise und oberflächlich demineralisierten Knochenpartikeln und Kombinationen davon bestehenden Gruppe ausgewählt sind.

27. Verfahren nach einem der Ansprüche 18 bis 26, **dadurch gekennzeichnet, dass** die länglichen demineralisierten Knochenpartikel ein Verhältnis von medianer Länge zu medianer Dicke von wenigstens 5:1 bis zu 500:1 aufweisen.

28. Verfahren nach einem der Ansprüche 18 bis 27, **dadurch gekennzeichnet, dass** das Netzmittel Wasser in Kombination mit einer oder mehreren Komponenten ausgewählt aus der aus organischen protischen Lösungsmitteln, physiologischer Kochsalzlösung, konzentrierten Kochsalzlösungen, Zuckerlösungen, ionischen Lösungen, flüssigen Polyhydroxyverbindungen und Polyoxyalkylenverbindungen bestehenden Gruppe enthält.

29. Verfahren nach einem der Ansprüche 18 bis 28, **dadurch gekennzeichnet, dass** das Netzmittel Wasser und Polyhydroxyverbindung enthält.

30. Verfahren nach Anspruch 29, **dadurch gekennzeichnet, dass** es sich bei der Polyhydroxyverbindung um Glycerin handelt.

31. Verfahren nach Anspruch 30, **dadurch gekennzeichnet, dass** das Gewichtsverhältnis von Glycerin zu Wasser im Bereich von 40:60 bis 5:95 liegt.

32. Verfahren nach einem der Ansprüche 18 bis 31, **dadurch gekennzeichnet, dass** das Osteoimplant nach dem Erhitzen in Schritt d einem weiteren Behandlungsschritt unterzogen wird.

33. Verfahren nach Anspruch 32, **dadurch gekennzeichnet, dass** der weitere Behandlungschritt aus der aus Lyophilisieren, Quervernetzen, Remineralisieren, Sterilisieren, maschineller Bearbeitung, Laserätzen, Schweißen, dem Zusammenbau von Teilen, Schneiden, Mahlen und reaktivem Ätzen bestehenden Gruppe ausgewählt ist.

34. Verfahren nach Anspruch 32, **dadurch gekennzeichnet, dass** es sich bei dem weiteren Behandlungsschritt um Lyophilisieren handelt.

35. Osteoimplant nach einem der Ansprüche 1 bis 34, **dadurch gekennzeichnet, dass** es wenigstens einen Hohlraum bzw. eine Vertiefung enthält.

36. Osteoimplant nach Anspruch 35, **dadurch gekennzeichnet, dass** der Hohlraum bzw. die Vertiefung ein fließfähiges osteogenes Material enthält.

37. Osteoimplant nach Anspruch 36, **dadurch gekennzeichnet, dass** das fließfähige Material aus der aus autologen Knochentransplantaten, Knochenmarkaspirat, demineralisierter Knochenmatrix und morphogenem Knochenprotein bestehenden Gruppe ausgewählt ist.

38. Verwendung eines Osteoimplants nach einem der Ansprüche 1 bis 37 zur Herstellung eines Osteoimplants, enthaltend eine geformte, kohärente dreidimensionale poröse Matrix aus länglichen demineralisierten Knochenpartikeln, **dadurch gekennzeichnet, dass** die Matrix eine Schüttdichte von weniger als 0,3 g/cm³ hat.

39. Verwendung nach Anspruch 38, **dadurch gekennzeichnet, dass** der reparierte Knochen aus der aus Siebbeinknochen, Stirnbeinknochen, Nasenknochen, Hinterhauptbein, Scheitelbein, Schläfenbein, Unterkieferknochen, Oberkieferknochen, Jochbein, Halswirbelknochen, Brustwirbelknochen, Lendenwirbelknochen, Kreuzbein, Rippenknochen, Brustbein, Schlüsselbein, Schulterblatt, Oberarmknochen, Speiche, Elle, Handwurzelknochen, Mittelhandknochen, Finger- und Zehenknochen, Darmbein, Sitzbein, Schambein, Oberschenkelknochen, Schienbein, Wadenbein, Kniescheibe, Fersenbein, Fußwurzelknochen und Mittelfußknochen bestehenden Gruppe ausgewählt ist.

40. Verwendung eines Osteoimplants nach einem der Ansprüche 1 bis 37 zum Einbringen des Osteoimplants zwischen benachbarten Wirbeln der Wirbelsäule.

41. Verwendung nach Anspruch 40, **dadurch gekennzeichnet, dass** das Osteoimplant wenigstens einen Hohlraum oder eine Vertiefung enthält, der/die wenigstens eine das Knochenwachstum induzierende Substanz enthält.

## Revendications

1. Implant osseux qui comprend une matrice façonnée, cohérente, poreuse, en trois dimensions de particules d'os déminéralisées allongées, dans lequel ladite matrice possède une densité apparente inférieure à 0,3 g/cm³.

2. Implant osseux selon la revendication 1 dans lequel la matrice contient en outre un ou plusieurs composants biocompatibles.

3. Implant osseux selon la revendication 2 dans lequel les composants biocompatibles sont choisis dans le groupe comprenant un liant, un agent de remplissage, une fibre, un plastifiant, un agent biostatique/biocide, un tensioactif, et une substance bioactive biocompatibles.

4. Implant osseux selon l'une quelconque des revendications 1 à 3 dans lequel les particules d'os allongées représentent au moins 50 pour-cent en poids de la matrice.

5. Implant osseux selon la revendication 4 dans lequel les particules d'os allongées représentent au moins 60 pour-cent en poids de la matrice.

6. Implant osseux selon la revendication 5 dans lequel les particules d'os allongées représentent au moins 90 pour-cent en poids de la matrice.

7. Implant osseux selon l'une quelconque des revendications 1 à 6 dans lequel la matrice comprend en outre des particules d'os possédant des dimensions autres qu'allongées.

8. Implant osseux selon l'une quelconque des revendications 1 à 7 dans lequel la matrice comprend en outre des particules d'os non déminéralisées.

9. Implant osseux selon l'une quelconque des revendications 1 à 8 dans lequel les particules d'os déminéralisées allongées sont choisies dans le groupe comprenant des particules d'os déminéralisées complètement, partiellement et superficiellement, et leurs associations.

10. Implant osseux selon l'une quelconque des revendications 1 à 9 dans lequel les particules d'os déminéralisées allongées possèdent un ratio longueur médiane sur épaisseur médiane d'au moins 5 : 1 à 500 : 1.

11. Implant osseux selon l'une quelconque des revendications 1 à 10 dans lequel la matrice comprend moins de 5 pour-cent en poids d'eau et est flexible.

12. Implant osseux selon l'une quelconque des revendications 1 à 11 dans lequel la matrice comprend moins de 5 pour-cent en poids d'eau et possède une capacité d'induction osseuse d'au moins 2 mesurée par test sur rat athymique.

13. Implant osseux selon l'une quelconque des revendications 1 à 12 dans lequel les particules d'os déminéralisées allongées sont réticulées.

14. Implant osseux selon l'une quelconque des revendications 1 à 13 dans lequel la densité apparente de l'implant osseux est de 0,01 à 0,3 g/cm³.

15. Implant osseux selon l'une quelconque des revendications 1 à 14 dans lequel la densité apparente de l'implant osseux est de 0,05 à 0,2 g/cm³.

16. Implant osseux selon l'une quelconque des revendications 1 à 15 en forme de feuille, de plaque, de disque, de cône, de broche, de vis, de tube, de dent, de racine dentaire, d'os ou de partie d'os, de coin ou de partie de coin, de cylindre et de cylindre fileté.

17. Implant osseux selon l'une quelconque des revendications 1 à 16, comprenant au moins une cavité ou dépression.

18. Procédé de fabrication d'un implant osseux qui comprend les étapes consistant à :
a) fournir une quantité de particules d'os déminéralisées allongées ;
b) mélanger les particules d'os déminéralisées allongées avec un agent mouillant comprenant de l'eau pour fournir une composition liquide contenant de 5 à 40 pour-cent en volume de particules d'os déminéralisées allongées gonflées hydratées ;
c) placer la composition liquide dans un moule ; et,
d) chauffer la composition liquide en absence sensible de pression à une température supérieure à 35 °C pendant une durée suffisante pour éliminer l'eau présente dans l'agent mouillant pour fournir un implant osseux comprenant une matrice poreuse façonnée, cohérente, en trois dimensions de particules d'os déminéralisées allongées, dans laquelle ladite matrice possède une densité apparente inférieure à 0,3 g/cm³.

19. Procédé selon la revendication 18 comprenant en outre l'incorporation d'un ou plusieurs composants biocompatibles dans la composition liquide.

20. Procédé selon la revendication 19 dans lequel les composants biocompatibles sont choisis dans le groupe comprenant un liant, un agent de remplissage, une fibre, un plastifiant, un agent biostatique/biocide, un tensioactif et une substance bioactive biocompatibles.

21. Procédé selon l'une quelconque des revendications 18 à 20 dans lequel les particules d'os déminéralisées allongées représentent au moins 50 pour-cent en poids de la matrice.

22. Procédé selon l'une quelconque des revendications 18 à 21 dans lequel les particules d'os déminéralisées allongées représentent au moins 60 pour-cent en poids de la matrice.

23. Procédé selon l'une quelconque des revendications 18 à 22 dans lequel les particules d'os déminéralisées allongées représentent au moins 90 pour-cent en poids de la matrice.

24. Procédé selon l'une quelconque des revendications 19 à 23 dans lequel le composant biocompatible comprend des particules osseuses possédant des dimensions autres qu'allongées.

25. Procédé selon l'une quelconque des revendications 19 à 24 dans lequel le composant biocompatible comprend des particules d'os non déminéralisées.

26. Procédé selon l'une quelconque des revendications 18 à 25 dans lequel les particules d'os déminéralisées allongées sont choisies dans le groupe comprenant des particules d'os déminéralisées complètement, partiellement et superficiellement, et leurs associations.

27. Procédé selon l'une quelconque des revendications 18 à 26 dans lequel les particules d'os déminéralisées allongées possèdent un ratio longueur médiane sur épaisseur médiane d'au moins 5 : 1 à 500 : 1.

28. Procédé selon l'une quelconque des revendications 18 à 27 dans lequel l'agent mouillant comprend de l'eau en association avec un ou plusieurs composants choisis dans le groupe comprenant un solvant protique organique, de la solution saline physiologique, des solutions salines concentrées, des solutions de sucre, des solutions ioniques, des composés polyhydroxy liquides, et des composés polyoxyalkylène.

29. Procédé selon l'une quelconque des revendications 18 à 28 dans lequel l'agent mouillant comprend de l'eau et un composé polyhydroxy.

30. Procédé selon la revendication 29 dans lequel le composé polyhydroxy est le glycérol.

31. Procédé selon la revendication 30 dans lequel le ratio en poids du glycérol sur l'eau est de 40 : 60 à 5 : 95.

32. Procédé selon l'une quelconque des revendications 18 à 31 dans lequel, après l'étape de chauffage d, l'implant osseux est soumis à une étape de traitement supplémentaire.

33. Procédé selon la revendication 32 dans lequel l'étape de traitement supplémentaire est choisie dans le groupe comprenant la lyophilisation, la réticulation, la re-minéralisation, la stérilisation, l'usinage, la gravure au laser, la soudure, l'assemblage d'éléments, la découpe, le broyage et la gravure réactive.

34. Procédé selon la revendication 32 dans lequel l'étape de traitement supplémentaire est la lyophilisation.

35. Implant osseux selon l'une quelconque des revendications 1 à 34 comprenant en outre une cavité ou dépression.

36. Implant osseux selon la revendication 35 dans lequel la cavité ou dépression contient un matériau ostéogène pouvant s'écouler.

37. Implant osseux selon la revendication 36 dans lequel le matériau ostéogène pouvant s'écouler est choisi dans le groupe comprenant un greffon osseux autologue, un aspirat de moelle osseuse, une matrice d'os déminéralisé et une protéine morphogénique osseuse.

38. Utilisation d'un implant osseux selon l'une quelconque des revendications 1 à 37 pour la fabrication d'un implant osseux qui comprend une matrice façonnée, cohérente, poreuse, en trois dimensions de particules d'os déminéralisées allongées, dans laquelle ladite matrice possède une densité apparente inférieure à 0,3 g/cm³.

39. Utilisation selon la revendication 38 dans laquelle l'os réparé est choisi dans le groupe comprenant l'os ethmoïde, frontal, nasal, occipital, pariétal, temporal, le mandibule, le maxillaire, l'os malaire, une vertèbre cervicale, une vertèbre thoracique, une vertèbre lombaire, le sacrum, une côte, le sternum, la clavicule, l'omoplate, l'humérus, le radius, le cubitus, les os du carpe, les os du métacarpe, les phalanges, l'ilion, l'ischion, le pubis, le fémur, le tibia, le péroné, la rotule, le calcanéum, les os du tarse et du métatarse.

40. Utilisation d'un implant osseux selon l'une quelconque des revendications 1 à 37 pour placer ledit implant osseux entre des vertèbres adjacentes de la colonne.

41. Utilisation selon la revendication 40 dans laquelle l'implant osseux comprend au moins une cavité ou dépression qui contient au moins une substance favorisant la croissance osseuse.
